# EUROPEAN PATENT APPLICATION

(11) **EP 3 482 772 A1**
(43) Date of publication of application: **15.05.2019**
(21) Application number: 17824076.8
(22) Date of filing: 27.06.2017
(51) Int. Cl.: A61K 39/395, A61K 45/06, A61P 35/00, A61P 35/02, A61P 37/04, A61P 43/00

(54) **ANTITUMOR AGENT**

(30) Priority: 05.07.2016 JP 2016133638
(71) Applicant: National University Corporation Kobe University, Kobe-shi, Hyogo 657-8501 (JP)
(72) Inventor: MATOZAKI, Takashi, Kobe-shi Hyogo 657-8501 (JP); MURATA, Yoji, Kobe-shi Hyogo 657-8501 (JP)
(74) Representative: LC Patents
(86) International application number: PCT/JP2017/023553
(87) International publication number: WO 2018/008470

(57) **Abstract**

Provided is an antitumor agent targeting SIRPα, which inhibits binding between CD47 and SIRPα, the antitumor agent being more effective. The present invention also provides an antitumor agent capable of more effectively exhibiting an antitumor effect when used in combination with an immune checkpoint inhibitor or an antibody drug. The antitumor agent includes as an active ingredient a substance that molecularly targets an IgV domain, which is an extracellular domain of SIRPα.The antitumor agent of the present invention, including as an active ingredient a substance that molecularly targets an IgV domain of SIRPα protein, activates M1-type macrophages, which have cytotoxicity to cancer cells, and immunocompetent cells to provide an effective antitumor effect. Further, the antitumor agent can effectively exhibit an antitumor action not only on cancer cells expressing SIRPα on a cell surface but also on cancer cells not expressing the SIRPα when used in combination with, for example, an immune checkpoint inhibitor and/or an antibody drug that specifically reacts with a cancer antigen and has ADCC and ADCP activities.

## Description

### Technical Field

The present invention relates to an antitumor agent including as an active ingredient a substance that molecularly targets an extracellular IgV domain of SIRPα protein. An excellent antitumor effect is obtained by the molecular targeting of the IgV domain.

The present application claims priority from Japanese Patent Application No. 2016-133638, which is incorporated herein by reference.

### Background Art

Signal regulatory protein α (SIRPα) is a protein belonging to an immunoglobulin superfamily including single-pass transmembrane receptor molecules, and has three Ig-like domains in its extracellular region and two immunoreceptor tyrosine-based inhibitory motifs (ITIMs) in its intracellular region. SIRPα is supposed to form a CD47-SIRPα system as an intercellular signaling system through an interaction with a five-pass transmembrane molecule CD47, which is a physiological ligand for its extracellular region, to thereby transduce signals bidirectionally. In addition, tyrosine phosphorylation of the ITIMs in the intracellular region of SIRPα is important for functions of SIRPα, and a tyrosine phosphatase SHP-2 or SHP-1 binds to SIRPα in a phosphorylation-dependent manner, which functions as a downstream signal of SIRPα.

In an immune system, SIRPα is strongly expressed in myeloid cells, such as dendritic cells and macrophages. Through analysis of genetically-modified mice for SIRPα, the inventors of the invention of the present application have already clarified that SIRPα is important for homeostasis of dendritic cells. In addition, the CD47-SIRPα system is also important for induction of Th17 cells important for autoimmune disease model development.

Usefulness of a molecularly-targeted drug, especially an antibody drug as a therapeutic method for cancer is beginning to be established. Cancer is still the leading cause of death, and development of a better therapeutic drug has been demanded. It is known that binding between CD47 and SIRPα suppresses an antitumor effect of immune cells. With attention focused on CD47, which is a ligand molecule for SIRPα, development of an antitumor agent that inhibits binding between CD47 and SIRPα is beginning to be advanced. There is a report that CD47 is found to be overexpressed in various types of cancers, and an anti-CD47 antibody has an antitumor effect on cancer transplanted into mice (Non Patent Literature 1: Proc Natl Acad Sci USA 109(17): 6662-6667. (2012)). There is also a report that combined use of the anti-CD47 antibody with an antibody drug having antibody-dependent-cellular cytotoxicity (ADCC) and/or antibody-dependent-cellular phagocytosis (ADCP) activity enhances drug efficacy of the antibody drug (Non Patent Literature 2: Cell 142(5): 699-713. (2010)). However, there are problems, for example, in that the antitumor effect of the anti-CD47 antibody is unknown for its mechanism of action, and that CD47 is ubiquitously expressed in all cells including cancer cells. That is, when the anti-CD47 antibody is used, there are risks of various side effects and toxicity. Meanwhile, it is unknown whether an anti-SIRPα antibody exhibits an antitumor effect when used alone or in combination with any other antibody drug.

There is a report of a receptor protein SHPS-1 (SH2-containing protein tyrosine phosphatase substrate-1) (synonym: SIRPα) belonging to the immunoglobulin superfamily as a dephosphorylation substrate protein for SHP-2 (Mol. Cell. Biol., 16: 6887-6899, 1996). There is a report of a pharmaceutical composition characterized by containing as an active ingredient an anti-SHPS-1 monoclonal antibody that specifically recognizes and binds to an N-terminal immunoglobulin-like structure of a dephosphorylation substrate protein SHPS-1 for an SH2 domain-containing protein together with a pharmacological ingredient (Patent Literature 1). In Patent Literature 1, there is a disclosure of an anti-human SHPS-1 monoclonal antibody (SE12C3) produced using a peptide SHPS-1 as an immunogen. In the literature, there is a disclosure that SE12C3 was found to exhibit a cell movement-suppressing effect on CHO-Ras cells expressing human SIRPα. In Patent Literature 1, there are disclosures of various anti-SHPS-1 monoclonal antibodies other than SE12C3. However, there is no disclosure of direct antitumor actions of the antibodies disclosed in Patent Literature 1, though there is a disclosure that the antibodies are important for the prevention or treatment of cancer cell metastasis and arteriosclerosis.

There is a report of a monoclonal antibody that inhibits binding between CD47 and SIRPα (Non Patent Literature 3). Also in Non Patent Literature 3, there is a disclosure of a monoclonal antibody (SE12C3), and there is also a disclosure that SE12C3 specifically acts on Ig1 of SIRPα1. It is conceivable that Ig1 of SIRPα1 corresponds to an extracellular domain IgV of SIRPα1. However, in Non Patent Literature 3, there is no disclosure of any antitumor action of SE12C3, though there is a disclosure that SE12C3 is involved in inhibition of binding between SIRPα on a cell surface and a recombinant protein CD47.

Many attempts have been made at immunotherapy as a therapeutic method for cancer. There is an action called an immune checkpoint serving as a brake on an immune action, such as binding between programmed cell death-1 (PD-1) and its ligand, programmed cell death-ligand 1 (PD-L1), on cytotoxic T cells, which attack cancer cells, and the action has attracted attention as a mechanism through which the cancer cells evade immune surveillance in recent years. Reactivation of the body's own immunity to cancer with an immune checkpoint inhibitor can provide an antitumor effect. Development of a pharmaceutical using an anti-PD-1 antibody or an anti-PD-L1 antibody as such immune checkpoint inhibitor has been advanced.

There is a demand for development of an antitumor agent that more effectively acts when used in combination with an immune checkpoint inhibitor and/or an antibody drug that has ADCC and ADCP activities and specifically reacts with a cancer antigen, such as rituximab, which is clinically applied as a specific antibody against CD20.

### Citation List

### Non Patent Literature

[NPL 1] Proc Natl Acad Sci USA 109(17): 6662-6667. (2012)
[NPL 2] Cell 142(5): 699-713. (2010)
[NPL 3] Blood 97(9): 2741-2749. (2001)

### Patent Literature

[PTL 1] JP 3914996 B2 (JP 2007-56037 A)

### Summary of Invention

### Technical Problem

It is an object of the present invention to provide an antitumor agent targeting SIRPα, which inhibits binding between CD47 and SIRPα, the antitumor agent being more effective. It is another object of the present invention to provide an antitumor agent capable of more effectively exhibiting an antitumor effect when used in combination with an immune checkpoint inhibitor and/or an antibody drug that specifically reacts with a cancer antigen and has ADCC and ADCP activities.

### Solution to Problem

An antibody against SIRPα, which is a membrane molecule, is considered to exhibit its effect through the following dual mechanism of action: (1) a direct action on a tumor, such as a cell-killing action or a cell movement-suppressing action, through induction of ADCC and ADCP activities by an anti-SIRPα antibody; and (2) such an action that the anti-SIRPα antibody inhibits binding between CD47 and SIRPα to be formed between phagocytes and cancer cells, and cancels a phagocytosis-suppressing action of a CD47-SIRPα system to enhance cytotoxic activity of the anti-SIRPα antibody itself. On the basis of those facts, the inventors of the invention of the present application have made extensive investigations on substances that inhibit binding between CD47 and SIRPα in order to achieve the above-mentioned objects. As a result, the inventors have found that a substance that molecularly targets an extracellular IgV domain of SIRPα enhances cell-mediated immunity associated with macrophages, natural killer cells (NK cells), or T cells to exhibit an antitumor effect. Thus, the inventors have completed the present invention. The inventors have also found that the substance more effectively exhibits an antitumor effect when used in combination with an immune checkpoint inhibitor or an antibody drug that has ADCC and ADCP activities and specifically reacts with a cancer antigen. The antitumor agent of the present invention can exhibit an antitumor effect not only on cancer cells expressing SIRPα but also on cancer cells not expressing the SIRPα.

That is, the present invention includes the following items.
1. An antitumor agent, including as an active ingredient an anti-SIRPα antibody that molecularly targets an extracellular IgV domain of SIRPα protein.
2. An antitumor agent according to the above-mentioned item 1, wherein the anti-SIRPα antibody enhances a phagocytic action of macrophages.
3. An antitumor agent according to the above-mentioned item 2, wherein the macrophages include M1-type macrophages.
4. An antitumor agent according to any one of the above-mentioned items 1 to 3, wherein the anti-SIRPα antibody includes any one of a monoclonal antibody, polyclonal antibodies, and an antibody fragment.
5. An antitumor agent according to any one of the above-mentioned items 1 to 4, wherein the antitumor agent further includes as the active ingredient an immune checkpoint inhibitor and/or an antibody drug that specifically reacts with a cancer antigen and has ADCC and ADCP activities, in addition to the anti-SIRPα antibody that molecularly targets an extracellular IgV domain of SIRPα protein.
6. An antitumor agent according to the above-mentioned item 5, wherein the immune checkpoint inhibitor includes any one selected from an inhibitor for binding between PD-L1 and PD-1, and a CTLA4 inhibitor.
7. An antitumor agent according to the above-mentioned item 5 or 6, wherein the antibody drug that specifically reacts with a cancer antigen and has ADCC and ADCP activities includes any one selected from an anti-CD20 antibody, an anti-HER2 antibody, and an anti-EGFR antibody.
8. An antitumor agent according to any one of the above-mentioned items 1 to 7, wherein the tumor includes one or a plurality of types of tumors selected from carcinoma, sarcoma, lymphoma, leukemia, myeloma, germinoma, brain tumor, carcinoid, neuroblastoma, retinoblastoma, and nephroblastoma.
9. An antitumor agent according to any one of the above-mentioned items 1 to 8, wherein the tumor includes one or a plurality of types of tumors selected from renal cell carcinoma, melanoma, squamous cell carcinoma, basal cell carcinoma, conjunctival cancer, oral cancer, laryngeal cancer, pharyngeal cancer, thyroid cancer, lung cancer, breast cancer, esophageal cancer, gastric cancer, duodenal cancer, small intestine cancer, colon cancer, rectal cancer, appendiceal cancer, anal cancer, hepatic cancer, gallbladder cancer, bile duct cancer, pancreatic cancer, adrenal cancer, bladder cancer, prostate cancer, uterine cancer, vaginal cancer, liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, epithelioid sarcoma, B-cell lymphoma, T-/NK-cell lymphoma, Hodgkin's lymphoma, myeloid leukemia, lymphoid leukemia, myeloproliferative disease, myelodysplastic syndrome, multiple myeloma, testicular cancer, ovarian cancer, glioma, and meningioma.
10. An agent for enhancing cell-mediated immunity, including as an active ingredient an anti-SIRPα antibody that molecularly targets an extracellular IgV domain of SIRPα protein.
11. An agent for enhancing cell-mediated immunity according to the above-mentioned item 10, wherein the cell-mediated immunity includes cell-mediated immunity associated with functional enhancement of natural killer cells and/or T cells.
   A. A therapeutic method for a tumor, including administering an anti-SIRPα antibody that molecularly targets an extracellular IgV domain of SIRPα protein.
   B. A therapeutic method according to the above-mentioned item A, wherein the anti-SIRPα antibody includes any one of a monoclonal antibody, polyclonal antibodies, and an antibody fragment.
   C. A therapeutic method for a tumor according to the above-mentioned item A or B, wherein the method includes further administering an immune checkpoint inhibitor and/or an antibody drug that specifically reacts with a cancer antigen and has ADCC and ADCP activities, in addition to the anti-SIRPα antibody that molecularly targets an extracellular IgV domain of SIRPα protein.
   D. A therapeutic method according to the above-mentioned item C, wherein the immune checkpoint inhibitor includes any one selected from an inhibitor for binding between PD-L1 and PD-1, and a CTLA4 inhibitor.
   E. A therapeutic method according to the above-mentioned item C or D, wherein the antibody drug that specifically reacts with a cancer antigen and has ADCC and ADCP activities includes any one selected from an anti-CD20 antibody, an anti-HER2 antibody, and an anti-EGFR antibody.
   F. A therapeutic method according to any one of the above-mentioned items A to E, wherein the tumor includes one or a plurality of types of tumors selected from carcinoma, sarcoma, lymphoma, leukemia, myeloma, germinoma, brain tumor, carcinoid, neuroblastoma, retinoblastoma, and nephroblastoma.
   G. A therapeutic method according to any one of the above-mentioned items A to F, wherein the tumor includes one or a plurality of types of tumors selected from renal cell carcinoma, melanoma, squamous cell carcinoma, basal cell carcinoma, conjunctival cancer, oral cancer, laryngeal cancer, pharyngeal cancer, thyroid cancer, lung cancer, breast cancer, esophageal cancer, gastric cancer, duodenal cancer, small intestine cancer, colon cancer, rectal cancer, appendiceal cancer, anal cancer, hepatic cancer, gallbladder cancer, bile duct cancer, pancreatic cancer, adrenal cancer, bladder cancer, prostate cancer, uterine cancer, vaginal cancer, liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, epithelioid sarcoma, B-cell lymphoma, T-/NK-cell lymphoma, Hodgkin's lymphoma, myeloid leukemia, lymphoid leukemia, myeloproliferative disease, myelodysplastic syndrome, multiple myeloma, testicular cancer, ovarian cancer, glioma, and meningioma.
   H. A method of enhancing cell-mediated immunity, including using an anti-SIRPα antibody that molecularly targets an extracellular IgV domain of SIRPα protein.
   I. A method of enhancing cell-mediated immunity according to the above-mentioned item H, wherein the cell-mediated immunity includes cell-mediated immunity associated with functional enhancement of natural killer cells and/or T cells.

### Advantageous Effects of Invention

The antitumor agent of the present invention, including as an active ingredient a substance that molecularly targets an extracellular IgV domain of SIRPα protein, activates M1-type macrophages, which have cytotoxicity to cancer cells, and other immune cells to provide an effective antitumor effect. In particular, the antitumor agent can be expected to exhibit an excellent antitumor effect not only on cancer cells expressing SIRPα but also on cancer cells not expressing the SIRPα when used in combination with, for example, an immune checkpoint inhibitor or an antibody drug that specifically reacts with a cancer antigen and has ADCC and ADCP activities.

### Brief Description of Drawings

FIGS. 1 are photographs for confirming target sites of anti-mouse SIRPα antibodies to be used in Examples in this description (Reference Example 1).
FIGS. 2A are photographs for confirming the expression of SIRPα in a tumor portion of the kidney of a patient with renal cell carcinoma by immunohistochemical staining, and FIGS. 2B are images for confirming the expressions of SIRPα in human renal cell carcinoma-derived cell lines (Reference Example 2).
FIG. 3 is a graph for comparing the expression levels of SIRPα mRNA in a normal tissue portion and a tumor portion of the kidney of a patient with renal cell carcinoma (Reference Example 2).
FIGS. 4A are photographs for confirming the expressions of a marker for melanoma and SIRPα in a tumor portion of a patient with melanoma by immunohistochemical staining, and FIGS. 4B are images for confirming the expressions of SIRPα in human melanoma-derived cell lines (Reference Example 2).
FIGS. 5 are photographs and a graph for showing the actions of anti-mouse SIRPα antibodies on aggregation between cells expressing mouse SIRPα and cells expressing mouse CD47 (Example 1).

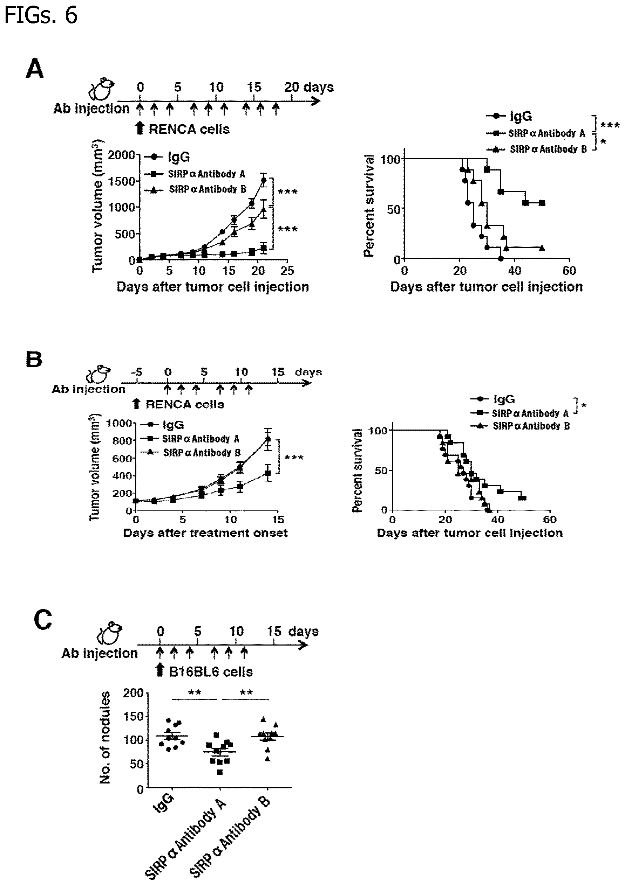

FIGS. 6 are graphs for showing the antitumor effects of anti-mouse SIRPα antibodies on mice transplanted with mouse renal cell carcinoma or mouse melanoma cells. FIGS. 6A are graphs for showing effects in the case where mice transplanted with RENCA cells (mouse renal cell carcinoma) were administered the anti-mouse SIRPα antibodies from the day of the transplantation of the RENCA cells, and FIGS. 6B are graphs for showing effects in the case where the mice transplanted with RENCA cells were administered the anti-mouse SIRPα antibodies from the time point when the tumor volume had achieved about 100 mm³. FIG. 6C is a graph for showing the effects of the anti-mouse SIRPα antibodies on mice transplanted with B16BL6 cells (mouse melanoma) (Example 2).
FIG. 7 is a graph for confirming the influence of the presence or absence of macrophages on the suppression of an increase in tumor volume by anti-mouse SIRPα antibody administration in mice transplanted with RENCA cells (Example 3).
FIGS. 8 are graphs for confirming the actions of anti-mouse SIRPα antibodies on macrophage phagocytic abilities for RENCA cells (Example 4).
FIGS. 9 are graphs for confirming proportions of macrophages and various immune cells present in tumors in mice transplanted with RENCA cells in the case where the mice were administered an anti-mouse SIRPα antibody (Example 5).
FIGS. 10 are graphs for confirming the influence of the depletion of specific immune cells on the suppression of an increase in tumor volume by anti-mouse SIRPα antibody administration in mice transplanted with RENCA cells (Example 6).
FIG. 11 is a graph for showing the suppression of an increase in tumor volume through the combined use of an anti-mouse SIRPα antibody and an immune checkpoint inhibitor in mice transplanted with CT26 cells (mouse colon cancer) not expressing SIRPα (Example 7).
FIGS. 12 are graphs for showing an antitumor effect through the combined use of an anti-mouse SIRPα antibody and an anti-CD20 antibody in Raji cells (human Burkitt's lymphoma) not expressing SIRPα (Example 8).
FIG. 13 is a graph for showing the suppression of an increase in tumor volume through the combined use of an anti-mouse SIRPα antibody and an immune checkpoint inhibitor in mice transplanted with RENCA cells (Example 9).
FIG. 14 is a graph for confirming a macrophage phagocytic ability for Raji cells (human Burkitt's lymphoma) through the combined use of an anti-human SIRPα antibody and an anti-CD20 antibody in mice expressing human SIRPα (Example 10).
FIG. 15 is a graph for showing an antitumor effect through the combined use of an anti-human SIRPα antibody and an anti-CD20 antibody in immunodeficient mice transplanted with Raji cells (human Burkitt's lymphoma) and expressing human SIRPα (Example 11).
FIGS. 16 are photographs for confirming a target site of the anti-human SIRPα antibody used in each of Examples 10 and 11 (Reference Example 3).

### Description of Embodiments

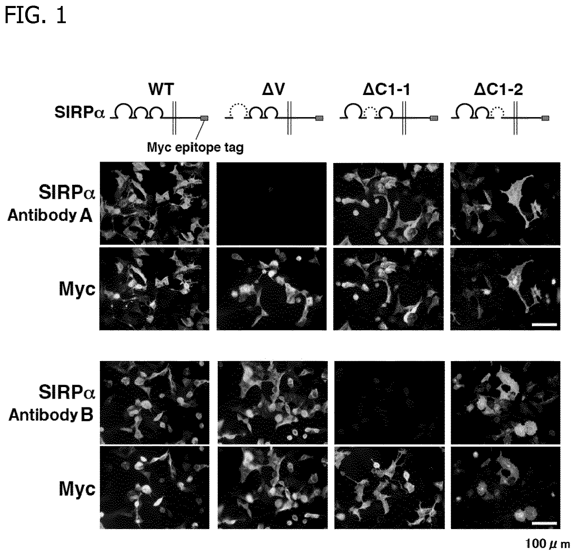

The present invention relates to an antitumor agent including as an active ingredient a substance that molecularly targets an extracellular IgV domain of SIRPα protein. The "extracellular IgV domain of SIRPα protein" of the present invention refers to an IgV domain, which is one of the three extracellular Ig-like domains constituting SIRPα. Also as shown in the "Background Art" section, the SIRPα protein belongs to an immunoglobulin superfamily including single-pass transmembrane receptor molecules, and has three Ig-like domains in its extracellular region and two ITIMs in its intracellular region (see FIGS. 1). The SIRPα protein in the present invention may be specified by, for example, each of GenBank Accession Nos.: NP_001035111 (human) and NP_001277949 (mouse). The IgV domain may be specified by each of partial sequences of GenBank Accession Nos.: NP_00103511 (human) and NP_001277949 (mouse) described above (amino acids at position 43 to position 144 and amino acids at position 43 to position 145, respectively).

Herein, the "substance that molecularly targets an extracellular IgV domain of SIRPα protein" refers to a substance capable of interacting with the IgV domain. Examples of such substance may include an anti-SIRPα antibody targeting the IgV domain, a peptide targeting the IgV domain, and an antisense strand capable of suppressing the expression of the IgV domain. Of those, an anti-SIRPα antibody targeting the IgV domain is a most suitable example.

Herein, the anti-SIRPα antibody targeting an extracellular IgV domain of SIRPα protein may be an antibody targeting the IgV domain selected from anti-SIRPα antibodies each produced using SIRPα as an antigen, or may be produced using the IgV domain as an antigen. In addition, the anti-SIRPα antibody may be any antibody selected from, for example, a monoclonal antibody or polyclonal antibodies and a multi-specific antibody (e.g., bispecific antibody). The form of the antibody may be any of an intact antibody and an antibody fragment. However, an intact immunoglobulin including an Fc portion having an effector function is suitable.

Herein, definitions and production methods for the monoclonal antibody and the polyclonal antibodies may conform to, for example, definitions and production methods known per se. In addition, the antibody may be a monoclonal antibody or polyclonal antibodies to be produced by any method to be developed in the future. In addition, the multi-specific antibody (e.g., bispecific antibody) is not particularly limited as long as the antibody has a function of molecularly targeting an extracellular IgV domain of SIRPα protein. A known method per se or any method to be developed in the future is applicable to a production method for the multi-specific antibody.

The antibody in this description may be an intact antibody or an antibody fragment. The intact antibody in this description is an antibody including two antigen-binding regions and one Fc portion. It is preferred that the intact antibody have a functional Fc portion. The antibody fragment includes a part of an intact antibody, and the part preferably includes its antigen-binding region. Examples of the antibody fragment include Fab, Fab', F(ab)'₂, and Fv fragments, a diabody, a linear antibody, a single-chain antibody molecule, and a multi-specific antibody formed from an antibody fragment.

Regarding antibody-specifying sites, definitions to be recognized by a so-called person skilled in the art are applicable to the terms for the sites, such as Fab, Fab', F(ab)'₂, Fv, and Fc. For example, the Fab fragment refers to an antigen-binding fragment having a single antigen-binding site, and the F(ab)'₂ fragment is a fragment having two antigen-binding sites. The Fv fragment is a minimum antibody fragment having an antigen-recognizing antigen-binding site. The Fc portion defines the C-terminal region of an immunoglobulin heavy chain, and has an effector function. Examples of the effector function include binding to C1q, complement-dependent cytotoxicity (CDC), binding to Fc receptor, ADCC, phagocytosis, and down-regulation of a cell surface receptor (e.g., B cell receptor: BCR). Such effector function generally requires a combination of the Fc portion with a binding domain (e.g., antibody variable domain).

The ADCC refers to a cell-mediated reaction in which non-specific cytotoxic cells (e.g., NK cells, neutrophils, and macrophages) expressing Fc receptor recognize an antibody bound to target cells, and thereafter, cause the lysis of the target cells. FcγRIIC and FcγRIIIA are expressed in NK cells, which are primary cells responsible for the ADCC, and FcyRI, FcyRIIA, FcyRIIC, and FcγRIIIA are expressed in monocytes. Meanwhile, the ADCP refers to a cell-mediated reaction in which phagocytes (e.g., macrophages and neutrophils) expressing Fc receptor recognize an antibody bound to target cells, and thereafter, phagocytose the target cells. FcyRI, FcyRIIA, FcyRIIC, and FcγRIIIA are expressed in monocytes, which are primary cells responsible for the ADCP.

The antitumor agent of the present invention, including as an active ingredient a substance that molecularly targets an extracellular IgV domain of SIRPα protein, may be used in combination with an immune checkpoint inhibitor and/or an antibody drug that specifically reacts with a cancer antigen and has ADCC and ADCP activities. Alternatively, the antitumor agent of the present invention may further include, in addition to the substance that molecularly targets the IgV domain, an immune checkpoint inhibitor and/or an antibody drug that specifically reacts with a cancer antigen and has ADCC and ADCP activities. Herein, examples of the immune checkpoint inhibitor include an inhibitor for binding between PD-1 and its ligand PD-L1, and a CTLA4 inhibitor. More specific examples thereof include an anti-PD-1 antibody (nivolumab or pembrolizumab), an anti-PD-L1 antibody (atezolizumab), and an anti-CTLA4 antibody (ipilimumab). In addition, examples of the antibody drug that specifically reacts with a cancer antigen and has ADCC and ADCP activities include an anti-CD20 antibody (rituximab), an anti-HER2 antibody (trastuzumab), and an anti-EGFR antibody (cetuximab).

The antitumor agent of the present invention may be used for one or a plurality of types selected from carcinoma, sarcoma, lymphoma, leukemia, myeloma, germinoma, brain tumor, carcinoid, neuroblastoma, retinoblastoma, and nephroblastoma. Specifically, examples of the carcinoma include renal cell carcinoma, melanoma, squamous cell carcinoma, basal cell carcinoma, conjunctival cancer, oral cancer, laryngeal cancer, pharyngeal cancer, thyroid cancer, lung cancer, breast cancer, esophageal cancer, gastric cancer, duodenal cancer, small intestine cancer, colon cancer, rectal cancer, appendiceal cancer, anal cancer, hepatic cancer, gallbladder cancer, bile duct cancer, pancreatic cancer, adrenal cancer, bladder cancer, prostate cancer, uterine cancer, and vaginal cancer. Examples of the sarcoma include liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, and epithelioid sarcoma. Examples of the lymphoma include B-cell lymphoma, T-/NK-cell lymphoma, and Hodgkin's lymphoma. Examples of the leukemia include myeloid leukemia, lymphoid leukemia, myeloproliferative disease, and myelodysplastic syndrome. An example of the myeloma is multiple myeloma. Examples of the germinoma include testicular cancer and ovarian cancer. Examples of the brain tumor include glioma and meningioma.

The substance that molecularly targets an extracellular IgV domain of SIRPα protein of the present invention may also be used as an agent for enhancing cell-mediated immunity. The cell-mediated immunity may be enhanced along with functional enhancement of NK cells and/or T cells.

The antitumor agent of the present invention may include a pharmaceutically acceptable carrier together with the substance that molecularly targets an extracellular IgV domain of SIRPα protein serving as the active ingredient. The "pharmaceutically acceptable carrier" may be appropriately selected from a wide range depending on the type of a target disease and the dosage form of a pharmaceutical agent. An administration method for the antitumor agent of the present invention may be appropriately selected. For example, the antitumor agent may be administered by injection, and local infusion, intraperitoneal administration, selective intravenous infusion, intravenous injection, subcutaneous injection, organ perfusate infusion, or the like may be adopted. In addition, a solution for injection may be formulated using a carrier formed of a salt solution, a glucose solution, or a mixture of salt water and a glucose solution, various buffers, and the like. Alternatively, the solution for injection may be prepared by mixing a formulation in a powder state with the liquid carrier upon use.

Other administration methods may also be appropriately selected along with the development of a formulation. In the case of oral administration, for example, an oral solution, a powder, a pill, a capsule, and a tablet are applicable. The oral solution may be produced by using, as an oral liquid adjusting agent, such as a suspension and a syrup, for example: sugars, such as water, sucrose, sorbitol, and fructose; glycols, such as polyethylene glycol; oils, such as sesame oil and soybean oil; antiseptic agents, such as an alkyl p-hydroxybenzoate; and flavors, such as a strawberry flavor and peppermint. The powder, the pill, the capsule, and the tablet may be formulated with, for example: an excipient, such as lactose, glucose, sucrose, or mannitol; a disintegrant, such as starch or sodium alginate; a lubricant, such as magnesium stearate or talc; a binder, such as polyvinyl alcohol, hydroxypropyl cellulose, or gelatin; a surfactant, such as a fatty acid ester; or a plasticizer, such as glycerin. The tablet and the capsule are preferred unit dosage forms in the composition of the present invention in terms of ease of administration. A solid carrier for manufacture is used in the manufacture of the tablet and the capsule.

### Examples

The present invention is described in detail below by way of Reference Examples and Examples for a better understanding of the present invention. However, the present invention is by no means limited to these Examples.

### (Reference Example 1) Preliminary Investigations on Anti-mouse SIRPα Antibodies

In this Reference Example, the results of preliminary investigations leading to the completion of the present invention are shown. In this Reference Example, the characteristics of two types of anti-mouse SIRPα rat monoclonal antibodies, i.e., an SIRPα antibody A and an SIRPα antibody B were confirmed. The SIRPα antibody A is an anti-mouse SIRPα rat monoclonal antibody disclosed in J Immunol., 187(5): 2268-2277 (2011), and the SIRPα antibody B is an anti-mouse SIRPα rat monoclonal antibody disclosed in Dev. Biol., 137(2): 219-232 (1990). HEK293A cells (human embryonic kidney cells) were transfected to express wild-type SIRPα, ΔV SIRPα (IgV domain-deficient SIRPα), ΔC1-1 SIRPα (IgC1-1 domain-deficient SIRPα), and ΔC1-2 SIRPα (IgC1-2 domain-deficient SIRPα), and the respective cells were subjected to immunostaining using the SIRPα antibody A or the SIRPα antibody B and an anti-Myc mouse monoclonal antibody (9E10) as primary antibodies and using fluorescently-labeled anti-rat and anti-mouse IgG antibodies as secondary antibodies to investigate the reactivities of the SIRPα antibody A and the SIRPα antibody B. As a result, it was found that the SIRPα antibody A was an antibody against an extracellular IgV domain of SIRPα, and the SIRPα antibody B was an antibody against an extracellular IgC1-1 domain of SIRPα (FIGS. 1). The expressions of wild-type SIRPα and each mutant form in the HEK293A cells were confirmed on the basis of the reactivity of the anti-Myc mouse monoclonal antibody (FIGS. 1). In Examples shown below, investigations were performed using the above-mentioned anti-mouse SIRPα rat monoclonal antibodies.

### (Reference Example 2) Confirmation of Expressions of SIRPα in Human Renal Cell Carcinoma and Human Melanoma

In this Reference Example, the expressions of SIRPα in human renal cell carcinoma and human melanoma were confirmed.

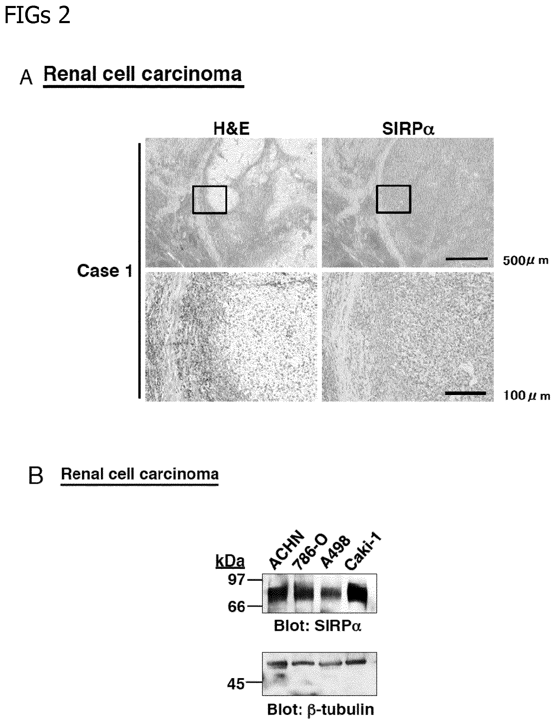

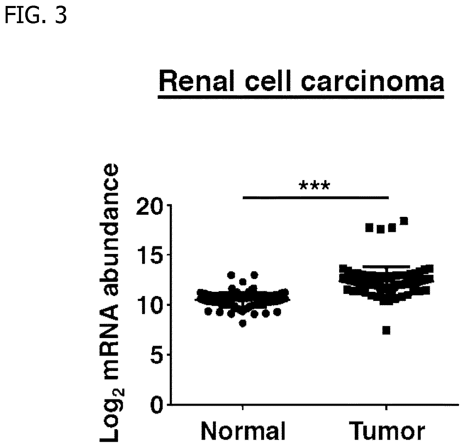

Paraffin-embedded sections of a kidney tissue including a tumor portion and a normal tissue portion of a patient with renal cell carcinoma were subjected to hematoxylin eosin (H&E) staining and immunostaining with anti-human SIRPα antibodies. As a result, strong staining with the anti-human SIRPα antibodies was found in the tumor portion, and high expression of SIRPα was found in renal cell carcinoma cells (FIGS. 2A). In addition, western blot analysis using protein lysates from human renal cell carcinoma-derived cell lines (ACHN, 786-O, A498, and Caki-1) and anti-human SIRPα antibodies revealed that SIRPα was expressed in the respective cell lines (FIGS. 2B). Western blotting with an anti-β-tubulin antibody revealed that a protein amount was constant among the protein lysates from the respective cell lines used (FIGS. 2B). Further, when the expression levels of SIRPα mRNA in a normal tissue portion and a tumor portion of the kidney of a patient with renal cell carcinoma were compared to each other, significantly high expression of SIRPα mRNA was found in the tumor portion (FIG. 3).

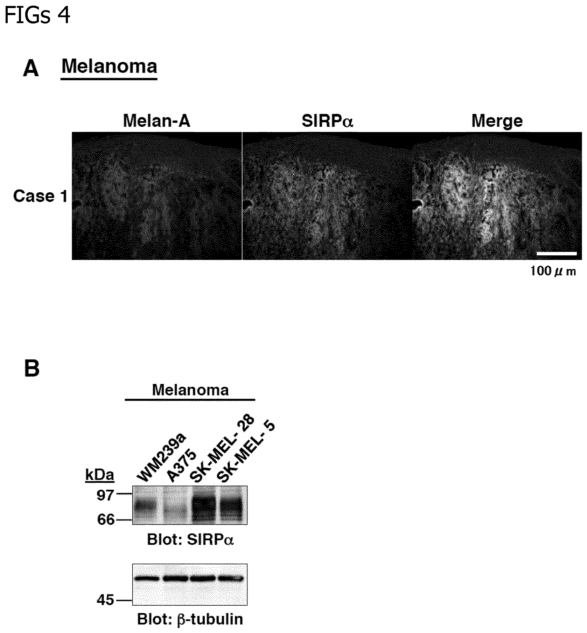

Frozen sections of a tissue including a tumor portion of a patient with melanoma were subjected to immunostaining with melan-A serving as a marker for melanoma. The sections were also subjected to immunostaining with an anti-human SIRPα antibody, and the stained images were merged. As a result, the expressions of melan-A and SIRPα were found at the same site of the tissue, and hence it was found that SIRPα was expressed in melanoma (FIGS. 4A). Further, western blot analysis using protein lysates from human melanoma-derived cell lines (WM239a, A375, SK-MEL-28, and SK-MEL-5) and anti-human SIRPα antibodies revealed that SIRPα was expressed in the respective cell lines (FIGS. 4B). Western blotting using an anti-β-tubulin antibody revealed that a protein amount was constant among the protein lysates from the respective cell lines used (FIGS. 4B).

### (Example 1) Actions of Anti-mouse SIRPα Antibodies on Aggregation between Cells expressing Mouse SIRPα and Cells expressing Mouse CD47

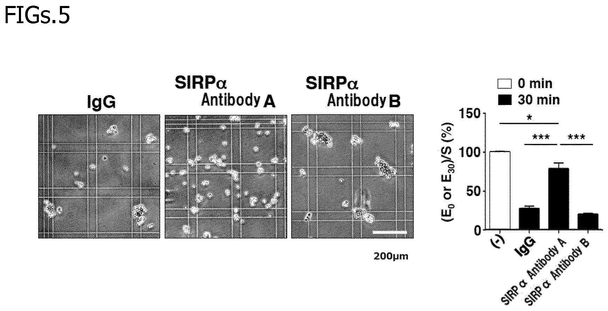

In this Example, the actions of two types of anti-mouse SIRPα antibodies, i.e., the SIRPα antibody A and the SIRPα antibody B on aggregation between CHO-Ras cells expressing mouse SIRPα and CHO-Ras cells expressing mouse CD47 were confirmed. Herein, the CHO-Ras cells refer to CHO cells expressing an active form of human Ras. Normal rat IgG was used as control IgG (IgG). CHO-Ras cells expressing mouse SIRPα and CHO-Ras cells expressing mouse CD47 pretreated with each antibody were mixed and subjected to a reaction for 30 minutes. As a result, it was found that the cells aggregated for the control IgG and the SIRPα antibody B, whereas cell aggregation was suppressed for the SIRPα antibody A (FIGS. 5). *P<0.05, ***P<0.001.

### (Example 2) Antitumor Effects of Anti-mouse SIRPα Antibodies in Mice transplanted with Mouse Renal Cell Carcinoma and Mouse Melanoma Cells

In this Example, in the case where mice were transplanted with RENCA cells (mouse renal cell carcinoma) or B16BL6 cells (mouse melanoma), the actions of each antibody, i.e., the SIRPα antibody A, the SIRPα antibody B, or control IgG (IgG) on the respective cancer cells were confirmed. In addition, survival rates were also confirmed in the mice transplanted with RENCA cells. SIRPα is expressed in the respective cells.
2-1. RENCA cells (5×10⁵ cells) were subcutaneously transplanted into 8-week-old BALB/c mice (n=8 per group). The mice were intraperitoneally administered each antibody (dose: 200 µg) 3 times a week from the day of the transplantation of the RENCA cells according to the administration schedule indicated in FIGS. 6A. As a result, an increase in tumor volume was found after the transplantation in the group receiving the SIRPα antibody B or the control IgG (IgG), whereas the suppression of an increase in tumor volume was found in the group receiving the SIRPα antibody A. The survival rates of the mice were also significantly excellent in the group receiving the SIRPα antibody A (FIGS. 6A). *P<0.05, ***P<0.001.
2-2. RENCA cells (5×10⁵ cells) were subcutaneously transplanted into 8-week-old BALB/c mice (n=11 per group). From the day of the transplantation of the RENCA cells, the mice were intraperitoneally administered each antibody (dose: 400 µg) 3 times a week from the time point when the average tumor volume had achieved 100 mm³ (day 5 after the transplantation) according to the administration schedule indicated in FIGS. 6B. As a result, an increase in tumor volume was found after the transplantation in the group receiving the SIRPα antibody B or the control IgG (IgG), whereas the suppression of an increase in tumor volume was found after the onset of the antibody administration in the group receiving the SIRPα antibody A (FIGS. 6B). In addition, the survival rates of the mice were also significantly excellent in the group receiving the SIRPα antibody A as compared to the group receiving the control IgG. *P<0.05, ***P<0.001.
2-3. B16BL6 cells (0.5×10⁵ cells) were intravenously transplanted into 8-week-old C57BL/6 mice via the tail vein (n=10 per group). After the transplantation of the B16BL6 cells, the mice were intraperitoneally administered each antibody (dose: 200 µg) 3 times a week according to the administration schedule indicated in FIG. 6C. As a result, the number of tumor nodules formed in the lungs was significantly small in the group receiving the SIRPα antibody A (FIG. 6C). **P<0.01.

As a result of this Example, in the cancer cells expressing SIRPα, it was found that the increase in tumor volume was suppressed by the administration of the SIRPα antibody A alone, and the survival rates of the mice after the transplantation of the cancer cells were also excellent. It was found that the SIRPα antibody A alone exhibited an antitumor effect on the cancer cells expressing SIRPα.

### (Example 3) Antitumor Effect of SIRPα Antibody A mediated by Macrophages in Mice transplanted with RENCA Cells

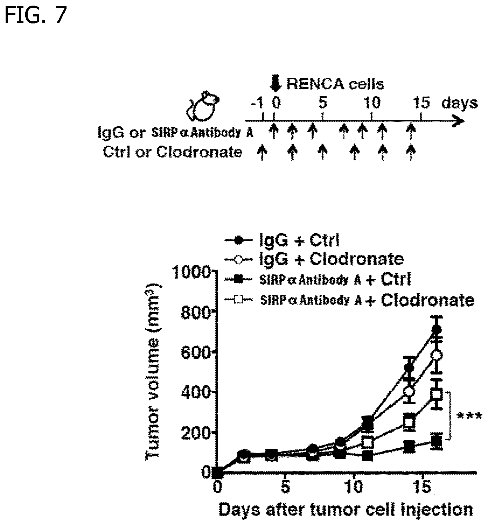

In the same manner as in 2-1 and 2-2 of Example 2, RENCA cells (5×10⁵ cells) were subcutaneously transplanted into 8-week-old BALB/c mice (n=8 per group). According to the administration schedule indicated in FIG. 7, the mice were intravenously administered clodronate-encapsulated liposomes (200 µl) 1 day before the transplantation of the RENCA cells as well as 100 µl of the liposomes every 3 days thereafter via the tail vein to deplete F4/80⁺CD11b⁺ macrophages from living bodies of the mice. The mice were administered phosphate-buffered saline-encapsulated liposomes as a control for clodronate. After the transplantation of the RENCA cells, the mice were intraperitoneally administered each antibody, i.e., the SIRPα antibody A or control IgG (IgG) (dose: 200 µg) 3 times a week according to the administration schedule indicated in FIG. 7. As a result, the strongest suppression of an increase in tumor volume was found in the group that received the SIRPα antibody A and was not subjected to the depletion of the F4/80⁺CD11b⁺ macrophages. Meanwhile, also in the groups subjected to the depletion of the F4/80⁺CD11b⁺ macrophages, the suppression of an increase in tumor volume was found, though slightly, in the group receiving the SIRPα antibody A as compared to the group receiving the control IgG. As a result of this Example, it was found that macrophages were involved in the antitumor effect of the administration of the SIRPα antibody A alone on the cancer cells expressing SIRPα in mouse individuals. ***P<0.001.

### (Example 4) Actions of Anti-mouse SIRPα Antibodies on Macrophage Phagocytic Ability (In Vitro System)

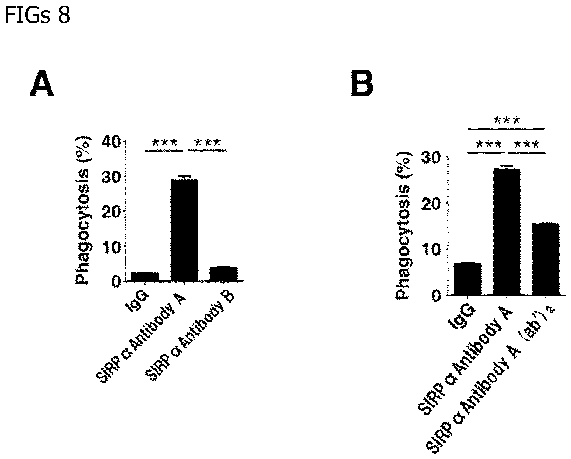

In this Example, the action of each antibody on the phagocytic ability of macrophages for RENCA cells was confirmed. Each antibody, i.e., the SIRPα antibody A, the SIRPα antibody B, or control IgG (IgG) was added (10 µg/ml) to carboxyfluorescein succinimidyl ester (CFSE)-labeled RENCA cells, and the cells were cultured at 37°C for 4 hours together with mouse bone marrow-derived macrophages. A phagocytic ability in this case was confirmed. The phagocytic ability was determined by quantifying the proportion of macrophages that had phagocytosed CFSE-positive cells in all macrophages. As a result, the strongest phagocytic ability was confirmed in the system having added thereto the SIRPα antibody A (FIG. 8A). Next, each antibody, i.e., the SIRPα antibody A, an SIRPα antibody A (Fab')₂, or control IgG was added by the same technique, and a phagocytic ability was confirmed. As a result, the strongest phagocytic ability was found in the system having added thereto the SIRPα antibody A, and a strong phagocytic ability was found, though inferior to that in the SIRPα antibody A system, in the SIRPα antibody A (Fab')₂ system as compared to the control IgG system (FIG. 8B). ***P<0.001.

As a result of this Example, it was found that the SIRPα antibody A was able to enhance the phagocytic ability of the macrophages on the cancer cells expressing SIRPα to exhibit ADCP activity.

### (Example 5) Influence of SIRPα Antibody A on Immune Cells in RENCA Tumors

In the same manner as in 2-1 and 2-2 of Example 2, RENCA cells (5×10⁵ cells) were subcutaneously transplanted into 8-week-old BALB/c mice (IgG group: n=6, SIRPα antibody A group: n=7 for evaluations of macrophages, NK cells, T cells, CD4⁺ T cells, and CD8⁺ T cells; IgG group: n=8, SIRPα antibody A group: n=9 for evaluations of myeloid-derived suppressor cells (MDSC) and regulatory T cells (Treg)). The mice were intraperitoneally administered each antibody, i.e., the SIRPα antibody A or control IgG (dose: 200 µg) from the day of the cell transplantation, and tumors formed under the skin were harvested on day 14. After that, the proportions of macrophages and various immune cells in CD45⁺ cells in the tumors were confirmed. The respective proportions are proportions of F4/80⁺Ly6C^{low} cells (macrophages), CD3ε⁻CD49b⁺ cells (NK cells), CD3ε⁺ cells (T cells), CD3ε⁺CD4⁺ cells (CD4⁺ T cells), CD3ε⁺CD8α⁺ cells (CD8⁺ T cells), CD11b⁺Gr-1⁺ cells (myeloid-derived suppressor cells), and CD3ε⁺CD4⁺Foxp3⁺ cells (suppressor T cells) in the CD45⁺ cells in the tumors.

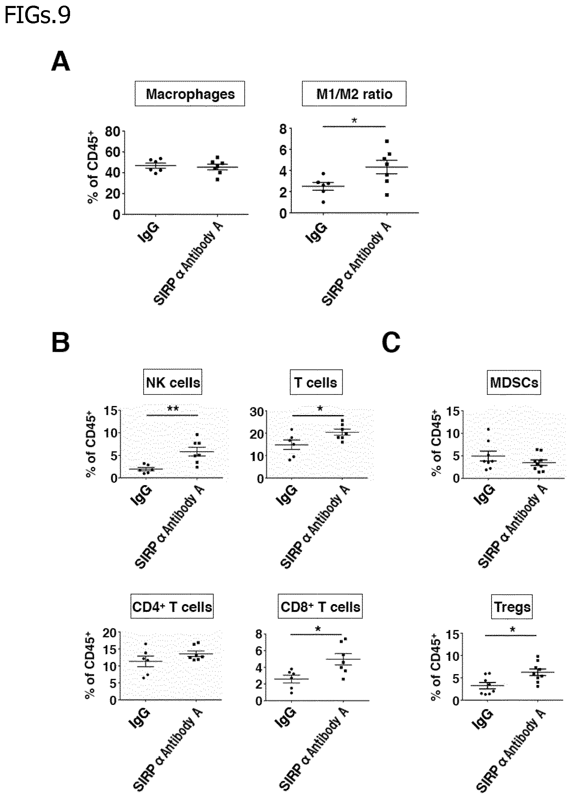

Macrophages are important cells that form a cancer microenvironment together with fibroblasts, vascular endothelial cells, and the like. The macrophages include M1-type and M2-type macrophages, and the M1-type macrophages are said to have cytotoxicity to cancer cells. When the above-mentioned antibody was administered, in the group receiving the SIRPα antibody A or the control IgG (IgG), the proportion of the macrophages in the CD45⁺ cells in the tumors was not changed. Meanwhile, when the ratio of the M1-type macrophages to the M2-type macrophages was confirmed, the ratio showed a higher value in the group receiving the SIRPα antibody A, and hence it was found that the proportion of the macrophages having high cytotoxicity to cancer cells was increased in the tumors (FIGS. 9A). As a result of this Example, it was found that the administration of the SIRPα antibody A to the cancer cells expressing SIRPα was able to increase the ratio of the M1-type macrophages, which had cytotoxicity to cancer cells, to the M2-type macrophages in the tumors of mouse individuals. *P<0.05.

As a result of confirmation for the NK cells and the T cells involved in tumor immunity, the proportions of both the cells in the CD45⁺ cells in the tumors showed significantly high values in the group receiving the SIRPα antibody A. Further, as a result of confirmation for the T cells, almost no difference was found in proportion of the CD4⁺ T cells between the groups receiving the SIRPα antibody A and the control IgG (IgG). However, the proportion of the CD8⁺ T cells showed a high value in the group receiving the SIRPα antibody A (FIGS. 9B). Meanwhile, for the myeloid-derived suppressor cells or the regulatory T cells known to be involved in the suppression of tumor immunity, no change was found in proportion of the myeloid-derived suppressor cells in the tumors in the group receiving the SIRPα antibody A or the control IgG, and an increase was found in proportion of the regulatory T cells in the group receiving the SIRPα antibody A as compared to the group receiving the control IgG (FIGS. 9C). *P<0.05, **P<0.01.

### (Example 6) Actions of NK Cells and T cells in Antitumor Effect of SIRPα Antibody A on RENCA Cells

In this Example, NK cells or CD8⁺ T cells were depleted from mice, and the action of each antibody on cancer cells was confirmed. The NK cells are depleted from mice by the administration of antibodies that recognize a glycolipid asialo-GM1 expressed on the cell surface of the NK cells. In addition, the CD8⁺ T cells are depleted from mice by the administration of an anti-CD8α antibody. Anti-asialo-GM1 rabbit polyclonal antibodies were used as the anti-asialo-GM1 antibodies, and an anti-mouse CD8α rat monoclonal antibody was used as the anti-CD8α antibody.

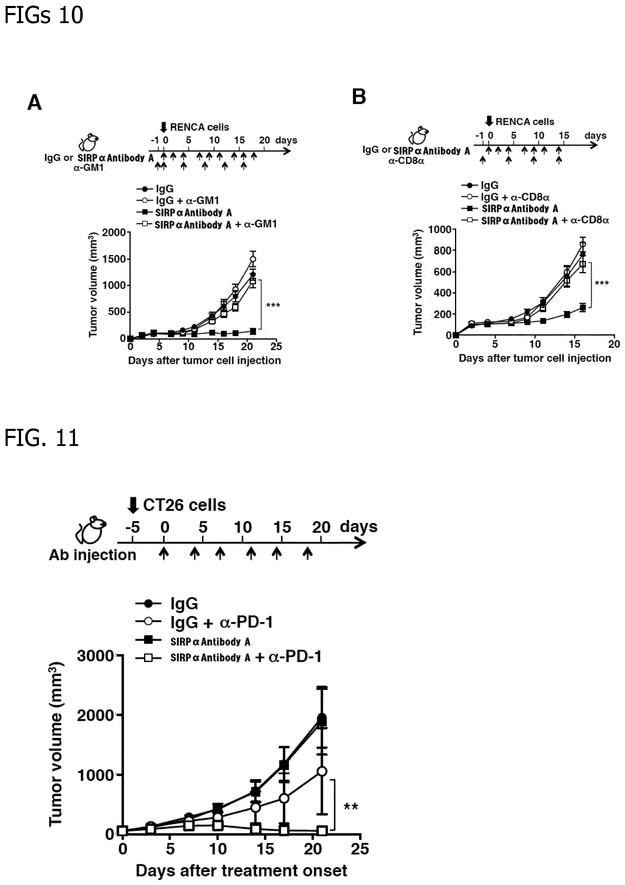

In the same manner as in Example 2, RENCA cells (5×10⁵ cells) were subcutaneously transplanted into 8-week-old BALB/c mice (n=10 per group). According to the administration schedule indicated in FIG. 10A, the mice were intraperitoneally administered the anti-asialo-GM1 antibodies (α-GM1, dose: 50 µl) 1 day before and on the day of the cell transplantation and then every 3 days thereafter. In addition, according to the administration schedule indicated in FIG. 10B, the mice were intraperitoneally administered the anti-CD8α antibody (α-CD8α, dose: 400 µg) 1 day before the cell transplantation and then every 5 days thereafter. Further, the mice were intraperitoneally administered the SIRPα antibody A or control IgG (IgG) 3 times a week from the day of the cell transplantation. As a result, when the NK cells or the CD8⁺ T cells were not depleted, clear suppression of an increase in tumor volume was found in the group receiving the SIRPα antibody A, whereas when the NK cells or the CD8⁺ T cells were depleted, almost no suppression of the increase was found in the group receiving the SIRPα antibody A, which was a comparable result to that of the group receiving the control IgG. This revealed that the antitumor effect of the SIRPα antibody A required the presence of the NK cells and the CD8⁺T cells responsible for immune response (FIG. 10A and FIG. 10B). ***P<0.001.

### (Example 7) Effect of Combined Use of SIRPα Antibody A and Immune Checkpoint Inhibitor (1)

In recent years, an immune checkpoint inhibitor, which cancels the suppression of the antitumor effect of CD8⁺ T cells, has attracted attention as a potent antitumor agent for various types of cancers. In view of the foregoing, it is conceivable that the combined use of the immune checkpoint inhibitor and the SIRPα antibody A can be expected to exhibit a more potent antitumor effect. Thus, in this Example, the antitumor effect of the combined use of the SIRPα antibody A and an anti-PD-1 antibody known as the immune checkpoint inhibitor was confirmed. An anti-mouse PD-1 rat monoclonal antibody was used as the anti-PD-1 antibody.

Mouse-derived colon cancer cells (CT26, 5×10⁵ cells) not expressing SIRPα were subcutaneously transplanted into 8-week-old BALB/c mice (n=6 per group). After the transplantation of the CT26 cells, the mice were intraperitoneally administered each antibody, i.e., the SIRPα antibody A, the anti-PD-1 antibody (α-PD-1), or control IgG (IgG) (dose: 100 µg) 3 times a week from the time point when the average tumor volume had achieved 100 mm³ (day 5 after the cell transplantation) according to the administration schedule indicated in FIG. 11. As a result, the suppression of an increase in tumor volume was found in the group receiving the anti-PD-1 antibody alone or receiving the combination of the anti-PD-1 antibody and the SIRPα antibody A, and higher suppression of the increase was found in the group receiving the combination. Meanwhile, almost no suppression of an increase in tumor volume was found in the group receiving the SIRPα antibody A alone or the group receiving the control IgG (FIG. 11). As a result of this Example, it was found that the SIRPα antibody A enhanced the antitumor effect of the anti-PD-1 antibody on the cancer cells not expressing SIRPα in mouse individuals. ***P<0.01.

### (Example 8) Effect of Combined Use of SIRPα Antibody A and Anti-CD20 Antibody

In this Example, the antitumor effect of the combined use of an anti-CD20 antibody (rituximab) and the SIRPα antibody A or the SIRPα antibody B was confirmed in terms of macrophage phagocytic ability and suppressive effect on an increase in tumor volume. The effect of the SIRPα antibody A or the SIRPα antibody B on cancer cells opsonized by the administration of the anti-CD20 antibody was confirmed. A synergistic effect through the combined use of the anti-CD20 antibody and the SIRPα antibody A or the SIRPα antibody B was confirmed on cancer cells in which ADCC and ADCP activities were induced. Rituxan (trademark) was used as the anti-CD20 antibody.

### 8-1. Effect on Macrophage Phagocytic Ability

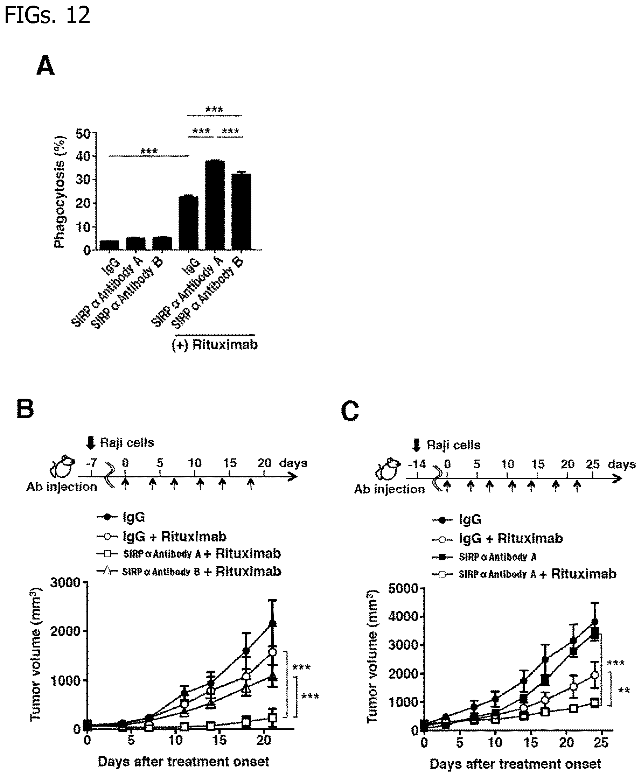

The anti-CD20 antibody and each antibody, i.e., the SIRPα antibody A, the SIRPα antibody B, or control IgG (IgG) were added to CFSE-labeled Raji cells (human Burkitt's lymphoma) (single agent: 10 µg/ml each; combined use: 5 µg/ml each), and the cells were cultured at 37°C for 4 hours together with bone marrow-derived macrophages harvested from non-obese diabetic (NOD) mice. The phagocytic ability of the macrophages for the Raji cells in this case was evaluated. The phagocytic ability was measured according to the method of Example 4. As a result, a significantly high phagocytic ability was found in the group receiving the combination of the anti-CD20 antibody and the SIRPα antibody A or the SIRPα antibody B as compared to the group receiving the combination of the anti-CD20 antibody and the control IgG. In addition, a significantly high phagocytic ability was found in the group receiving the combination of the anti-CD20 antibody and the SIRPα antibody A as compared to the group receiving the combination of the anti-CD20 antibody and the SIRPα antibody B (FIG. 12A). ***P<0.001.

### 8-2. Suppressive Effect on Increase in Tumor Volume (Treatment Onset on Day 7 after Cancer Cell Transplantation)

Raji cells (3×10⁶ cells) were subcutaneously transplanted into 6-week-old non-obese diabetic/severe combined immunodeficiency (NOD/SCID) mice serving as immunodeficient mice (n=5 per group). According to the administration schedule indicated in FIG. 12B, the mice were intraperitoneally administered the anti-CD20 antibody (dose: 40 µg) and each antibody, i.e., the SIRPα antibody A, the SIRPα antibody B, or control IgG (IgG) (dose: 200 µg) every 3 days from day 7 after the transplantation of the Raji cells to investigate the suppression of an increase in tumor volume. As a result, the strongest suppression of the increase in tumor volume was found in the group receiving the combination of the SIRPα antibody A and the anti-CD20 antibody (FIG. 12B). ***P<0.001.

### 8-3. Suppressive Effect on Increase in Tumor Volume (Treatment Onset on Day 14 after Cancer Cell Transplantation)

Raji cells (3×10⁶ cells) were subcutaneously transplanted into 6-week-old NOD/SCID mice (n=5 per group). According to the administration schedule indicated in FIG. 12C, the mice were intraperitoneally administered the anti-CD20 antibody (dose: 150 µg) and each antibody, i.e., the SIRPα antibody A, the SIRPα antibody B, or control IgG (dose: 200 µg) twice a week from day 14 after the transplantation of the Raji cells (time point when the tumor volume had achieved from 150 mm³ to 200 mm³) to investigate the suppression of an increase in tumor volume. As a result, no suppression of the increase in tumor volume was found in the group receiving the SIRPα antibody A or the control IgG (IgG) alone. Meanwhile, the suppression of tumor growth was found in the group receiving the anti-CD20 antibody alone, but the strongest suppression of the increase in tumor volume was found in the group receiving the combination of the SIRPα antibody A and the anti-CD20 antibody (FIG. 12C). **P<0.01, ***P<0.001.

As a result of this Example, it was found that the SIRPα antibody A enhanced the ADCP activity of the anti-CD20 antibody on the cancer cells, and the combined use of the SIRPα antibody A and the anti-CD20 antibody exhibited an excellent antitumor effect on the cancer cells in the mice as compared to the use of each of the anti-CD20 antibody and the SIRPα antibody A alone.

### (Example 9) Effect of Combined Use of SIRPα Antibody A and Immune Checkpoint Inhibitor (2)

In this Example, by the same technique as that of Example 7, the antitumor effect of the combined use of the SIRPα antibody A and an anti-PD-1 antibody was confirmed using mice subcutaneously transplanted with RENCA cells. An anti-mouse SIRPα antibody and an anti-mouse PD-1 rat monoclonal antibody were used as the SIRPα antibody A and the anti-PD-1 antibody, respectively.

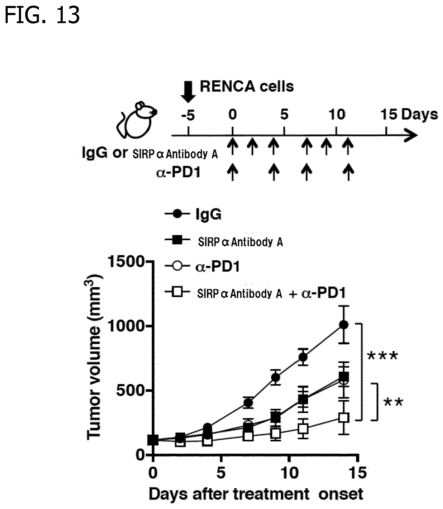

RENCA cells (5×10⁵ cells) were subcutaneously transplanted into 8-week-old BALB/c mice (group receiving a control IgG antibody: n=8; group receiving the anti-PD-1 antibody or the SIRPα antibody A alone and group receiving the combination of the anti-PD-1 antibody and the SIRPα antibody A: n=10). After the transplantation of the RENCA cells, the mice were intraperitoneally administered the anti-PD-1 antibody (α-PD-1, dose: 100 µg) and each antibody, i.e., the SIRPα antibody A or control IgG (dose: 200 µg) from the time point when the average tumor volume had achieved 100 mm³ according to the administration schedule indicated in FIG. 13, and the volumes of tumors formed under the skin were measured with time, to thereby investigate the antitumor effects of the various antibodies.

As a result, the combined use of the SIRPα antibody A and the anti-PD-1 antibody most strongly suppressed the increase in tumor volume, suggesting that the combined use exhibited a potent antitumor effect as compared to each antibody alone (FIG. 13). **P<0.01, ***P<0.001.

### (Example 10) Antitumor Action of Combined Use of Anti-human SIRPα Antibody and Anti-CD20 Antibody (1)

In this Example, the antitumor effect of the combined use of an anti-human SIRPα antibody and an anti-CD20 antibody (rituximab) was confirmed in terms of macrophage phagocytic ability and suppressive effect on an increase in tumor volume. An anti-human SHPS-1 monoclonal antibody (SE12C3) disclosed in Patent Literature 1 was used as the anti-human SIRPα antibody. It is described in Reference Example 3 below that the anti-human SHPS-1 monoclonal antibody (SE12C3) is an anti-SIRPα antibody that molecularly targets an extracellular IgV domain of SIRPα protein. In addition, Rituxan (trademark) was used as the anti-CD20 antibody in the same manner as in Example 8. The anti-human SIRPα antibody (SE12C3), the anti-CD20 antibody (rituximab), and control IgG (IgG) were added alone or in combination thereof to CFSE-labeled Raji cells, and the cells were cultured at 37°C for 4 hours together with bone marrow-derived macrophages prepared from immunodeficient mice expressing human SIRPα (mice disclosed in Proc Natl Acad Sci USA., 108(32): 13218-13223 (2011)). The phagocytic ability of the macrophages for the Raji cells in this case was evaluated. Doses were set as follows: 2.5 µg/ml of the anti-human SIRPα antibody (SE12C3) or the control IgG; and 0.025 µg/ml of the anti-CD20 antibody (rituximab). The phagocytic ability was measured according to the method of Example 4.

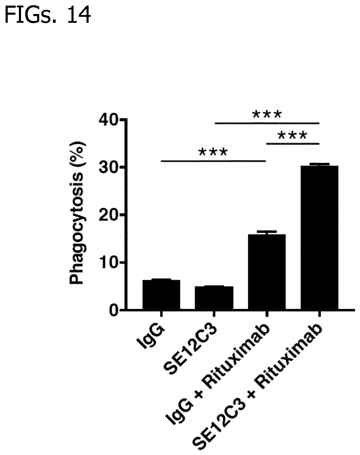

As a result, a significantly high phagocytic ability was found in the group receiving the combination of the anti-human SIRPα antibody and the anti-CD20 antibody as compared to the group receiving each antibody alone and the group receiving the combination of the control IgG and the anti-CD20 antibody (FIG. 14). ***P<0.001.

### (Example 11) Antitumor Action of Combined Use of Anti-human SIRPα Antibody and Anti-CD20 Antibody (2)

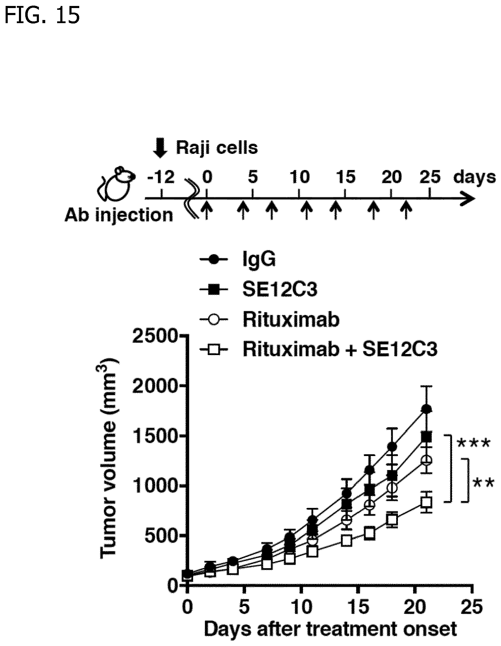

In this Example, an antitumor effect in the combined use of the anti-human SIRPα antibody (SE12C3) and the anti-CD20 antibody (rituximab) shown in Example 10 was confirmed. Raji cells (1×10⁶ cells) were subcutaneously transplanted into 6-week-old immunodeficient mice expressing human SIRPα (groups receiving the control IgG and the anti-human SIRPα antibody: n=10; group receiving the anti-CD20 antibody and group receiving the combination of the anti-human SIRPα antibody and the anti-CD20 antibody: n=12). After the transplantation of the Raji cells, the mice were intraperitoneally administered the anti-CD20 antibody (dose: 150 µg) and each antibody, i.e., the anti-human SIRPα antibody (SE12C3) or the control IgG (dose: 200 µg) from the time point when the tumor volume had achieved about 100 mm³ according to the administration schedule indicated in FIG. 15, and the volumes of tumors formed under the skin were measured with time, to thereby investigate the antitumor effects of the various antibodies.

As a result, the combined use of the anti-human SIRPα antibody and the anti-CD20 antibody most strongly suppressed the increase in tumor volume, suggesting that the combined use exhibited a potent antitumor effect as compared to each antibody alone (FIG. 15). **P<0.01, ***P<0.001.

### (Reference Example 3) Anti-human SIRPα Antibody

That the anti-human SIRPα antibody used in each of Examples 10 and 11 is an anti-SIRPα antibody that molecularly targets an extracellular IgV domain of SIRPα protein is described below.

As described above, the anti-human SIRPα antibody is the anti-human SHPS-1 monoclonal antibody (SE12C3) disclosed in Patent Literature 1. In Table. 1 in the left column on page 2744 of Non Patent Literature 3, there is a disclosure that the anti-human SHPS-1 monoclonal antibody (SE12C3) is a specific antibody against SIRPα1 IgG1.

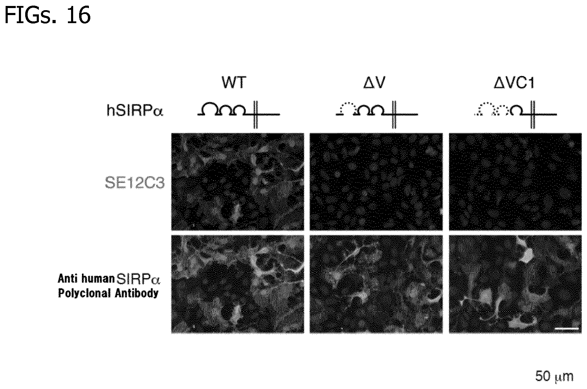

Further, by the same technique as that of Reference Example 1, the anti-human SHPS-1 monoclonal antibody (SE12C3) serving as the anti-human SIRPα antibody was confirmed for its molecular targeting site. HEK293A cells (human embryonic kidney cells) were transfected to express wild-type human SIRPα (WT), IgV domain-deficient human SIRPα (ΔV), and lgV/lgC1 domain-deficient human SIRPα (ΔVC1), and the respective cells were subjected to immunostaining using the above-mentioned anti-human SHPS-1 monoclonal antibody (SE12C3) and anti-human SIRPα polyclonal antibodies as primary antibodies and using a fluorescently-labeled anti-mouse IgG antibody or anti-rabbit IgG antibody as a secondary antibody to investigate the reactivities of the primary antibodies. The anti-human SHPS-1 monoclonal antibody (SE12C3) was found to be an antibody against an extracellular IgV domain of human SIRPα (hSIRPα) protein (FIGS. 16).

In Patent Literature 1, there is no disclosure of any direct tumor-suppressing action of the anti-human SHPS-1 monoclonal antibody (SE12C3), though there is a disclosure that SE12C3 is important for the prevention or treatment of cancer cell metastasis and arteriosclerosis. In Non Patent Literature 3, there is no disclosure of any action of SE12C3 on tumors or cancers. That is, the antitumor action of the anti-human SHPS-1 monoclonal antibody (SE12C3) serving as the anti-human SIRPα antibody, which was confirmed for the first time in the present invention, had not been known at all at the time of the publication of Patent Literature 1 and Non Patent Literature 3.

### Industrial Applicability

As described in detail above, the antitumor agent of the present invention, including as an active ingredient an antibody that molecularly targets an extracellular IgV domain of SIRPα protein, activates M1-type macrophages, which have cytotoxicity to cancer cells, and immunocompetent cells to provide an effective antitumor effect. In particular, the antitumor agent can be expected to exhibit an excellent antitumor effect not only on cancer cells expressing SIRPα but also on cancer cells not expressing the SIRPα when used in combination with, for example, an immune checkpoint inhibitor and/or an antibody drug that specifically reacts with a cancer antigen and has ADCC and ADCP activities.

The antitumor agent of the present invention can be expected to exhibit a more potent antitumor effect when used in combination with a chemotherapeutic agent or a radiotherapy. In addition, the antitumor agent can be expected to enhance one's own immunity to reduce the usage amount of the chemotherapeutic agent and the frequency of the radiotherapy, to thereby reduce side effects due to such therapy.

## Claims

1. An antitumor agent, comprising as an active ingredient an anti-SIRPα antibody that molecularly targets an extracellular IgV domain of SIRPα protein.

2. An antitumor agent according to claim 1, wherein the anti-SIRPα antibody enhances a phagocytic action of macrophages.

3. An antitumor agent according to claim 2, wherein the macrophages comprise M1-type macrophages.

4. An antitumor agent according to any one of claims 1 to 3, wherein the anti-SIRPα antibody comprises any one of a monoclonal antibody, polyclonal antibodies, and an antibody fragment.

5. An antitumor agent according to any one of claims 1 to 4, wherein the antitumor agent further comprises as the active ingredient an immune checkpoint inhibitor and/or an antibody drug that specifically reacts with a cancer antigen and has ADCC and ADCP activities, in addition to the anti-SIRPα antibody that molecularly targets an extracellular IgV domain of SIRPα protein.

6. An antitumor agent according to claim 5, wherein the immune checkpoint inhibitor comprises any one selected from an inhibitor for binding between PD-L1 and PD-1, and a CTLA4 inhibitor.

7. An antitumor agent according to claim 5 or 6, wherein the antibody drug that specifically reacts with a cancer antigen and has ADCC and ADCP activities comprises any one selected from an anti-CD20 antibody, an anti-HER2 antibody, and an anti-EGFR antibody.

8. An antitumor agent according to any one of claims 1 to 7, wherein the tumor comprises one or a plurality of types of tumors selected from carcinoma, sarcoma, lymphoma, leukemia, myeloma, germinoma, brain tumor, carcinoid, neuroblastoma, retinoblastoma, and nephroblastoma.

9. An antitumor agent according to any one of claims 1 to 8, wherein the tumor comprises one or a plurality of types of tumors selected from renal cell carcinoma, melanoma, squamous cell carcinoma, basal cell carcinoma, conjunctival cancer, oral cancer, laryngeal cancer, pharyngeal cancer, thyroid cancer, lung cancer, breast cancer, esophageal cancer, gastric cancer, duodenal cancer, small intestine cancer, colon cancer, rectal cancer, appendiceal cancer, anal cancer, hepatic cancer, gallbladder cancer, bile duct cancer, pancreatic cancer, adrenal cancer, bladder cancer, prostate cancer, uterine cancer, vaginal cancer, liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, epithelioid sarcoma, B-cell lymphoma, T-/NK-cell lymphoma, Hodgkin's lymphoma, myeloid leukemia, lymphoid leukemia, myeloproliferative disease, myelodysplastic syndrome, multiple myeloma, testicular cancer, ovarian cancer, glioma, and meningioma.

10. An agent for enhancing cell-mediated immunity, comprising as an active ingredient an anti-SIRPα antibody that molecularly targets an extracellular IgV domain of SIRPα protein.

11. An agent for enhancing cell-mediated immunity according to claim 10, wherein the cell-mediated immunity comprises cell-mediated immunity associated with functional enhancement of natural killer cells and/or T cells.
